# EUROPEAN PATENT SPECIFICATION

(11) **EP 2 140 028 B1**
(45) Date of publication and mention of the grant of the patent: **21.09.2011**
(21) Application number: 08718327.3
(22) Date of filing: 28.03.2008
(51) Int. Cl.: C12Q 1/68

(54) **METHOD OF NUCLEIC ACID ANALYSIS TO ANALYZE THE METHYLATION PATTERN OF CPG ISLANDS IN DIFFERENT SAMPLES**
NUKLEINSÄUREANALYSEVERFAHREN ZUR ANALYSE DES METHYLIERUNGSMUSTERS VON CPG-INSELN IN UNTERSCHIEDLICHEN PROBEN
PROCÉDÉS D'ANALYSE D'ACIDE NUCLÉIQUE POUR L'ANALYSE DU PROFIL DE MÉTHYLATION DES ILÔTS CPG DANS DIFFÉRENTS ÉCHANTILLONS

(30) Priority: 30.03.2007 ES 200700965
(43) Date of publication of application: 06.01.2010
(73) Proprietor: Oryzon Genomics, S.A., 08028 Barcelona (ES)
(72) Inventor: MAES, Tamara, E-08860 Castelldefels (Barcelona) (ES); DURANY TURK, Olga, E-08025 Barcelona (ES); AIBAR DURÁN, Elena, E-08840 Viladecans (Barcelona) (ES)
(74) Representative: ZBM Patents
(86) International application number: PCT/EP2008/053748
(87) International publication number: WO 2008/119765

(56) References cited:
- WO-A-03/027259
- US-A1- 2003 129 602
- US-A1- 2004 137 473
- US-A1- 2006 204 988
- HATADA I ET AL: "A microarray-based method for detecting methylated loci" JOURNAL OF HUMAN GENETICS, XX, XX, vol. 47, 1 January 2002 (2002-01-01), pages 448-451, XP002986571
- YAN P S ET AL: "Dissecting complex epigenetic alterations in breast cancer using CpG Island microarrays" CANCER RESEARCH, AMERICAN ASSOCIATION FOR CANCER RESEARCH, BALTIMORE, MD, vol. 61, no. 23, 1 December 2001 (2001-12-01), pages 8375-8380, XP002243660 ISSN: 0008-5472
- ADRIEN L R ET AL: "Classification of DNA methylation patterns in tumor cell genomes using a CpG island microarray" CYTOGENETIC AND GENOME RESEARCH, ALLERTON PRESS, NEW YORK, NY, US, vol. 114, no. 1, 1 January 2006 (2006-01-01), pages 16-23, XP009067710 ISSN: 1424-8581
- KHULAN BATBAYAR ET AL: "Comparative isoschizomer profiling of cytosine methylation: the HELP assay." GENOME RESEARCH AUG 2006, vol. 16, no. 8, August 2006 (2006-08), pages 1046-1055, XP002482920 ISSN: 1088-9051
- SMITH R J ET AL: "Identification of imprinted loci by methylation" METHODS IN MOLECULAR BIOLOGY, HUMANA PRESS INC., CLIFTON, NJ, US, vol. 181, 1 January 2001 (2001-01-01), pages 113-132, XP009067894 ISSN: 1064-3745
- FRIGOLA JORDI ET AL: "Methylome profiling of cancer cells by amplification of inter-methylated sites (AIMS)" NUCLEIC ACIDS RESEARCH, OXFORD UNIVERSITY PRESS, SURREY, GB, vol. 30, no. 7, 1 April 2002 (2002-04-01), pages e28/1-e28/7, XP002387724 ISSN: 0305-1048
- SCHONES DUSTIN E ET AL: "Genome-wide approaches to studying chromatin modifications." NATURE REVIEWS. GENETICS MAR 2008, vol. 9, no. 3, March 2008 (2008-03), pages 179-191, XP002482921 ISSN: 1471-0064

## Description

### FIELD OF THE INVENTION

The present invention relates to the field of molecular biology. In particular, the object of the present invention is a method of nucleic acid analysis that can be used to analyze the methylation pattern of CpG islands in different samples.

### BACKGROUND OF THE INVENTION

It has become clear in recent times that epigenetic factors can play a very important role in the genetic control of cellular processes, including the development of cancer. Among these epigenetic factors, methylation of particular DNA fragments is one of the most significant.

DNA methylation is an epigenetic process that is involved in regulating gene expression in two ways: directly, by preventing transcription factors from binding, and indirectly, by favoring the "closed" structure of chromatin (Singal R, & Ginder GD. DNA methylation. Blood. 1999 Jun 15;93(12):4059-70). DNA has regions of 1000-1500 bp rich in CpG dinucleotides (CpG islands), which are recognized by the DNA methyltransferases which, during DNA replication, methylate the carbon-5 position of cytosines in the recently synthesized string, so that the memory of the methylated state is preserved in the daughter DNA molecule. Methylation is generally considered to be a one-way process, so that when a CpG sequence is methylated de novo, this change becomes stable and is inherited as a clonal methylation pattern. Moreover, the change in the methylation state of regulatory genes (hypomethylation or hypermethylation), being a primary event, is frequently associated with the neoplastic process and is proportional to the severity of the disease (Paluszczak J, & Baer-Dubowska W. Epigenetic diagnostics of cancer - the application of DNA methylation markers. J Appl Genet. 2006;47(4):365-75).

The genomes of preneoplastic, cancerous, and aging cells share three important changes in methylation levels, marking them out as early events in the development of certain tumors. Firstly, hypomethylation of heterochromatin, leading to genomic instability and an increase in mitotic recombination events; secondly, hypermethylation of individual genes, and lastly, hypermethylation of the CpG islands of constitutive and tumor suppressor genes. The two methylation levels can occur separately or simultaneously; generally speaking, hypermethylation is involved in gene silencing and hypomethylation is involved in the overexpression of certain proteins implicated in the processes of invasion and metastasis.

Methodological strategies for analyzing the methylation state of CpG islands have been constantly evolving. Most of the methods are based on the chemical conversion of unmethylated cytosines to uracils by treating them with sodium bisulfite, which does not affect the 5-methylcytosines and individually and reliably identifies the CpG dinucleotides as being either methylated or unmethylated. DNA modification, its amplification by polymerase chain reaction (PCR), and/or automated sequencing are the most commonly used techniques in this context (Esteller M. Aberrant DNA methylation as a cancer-inducing mechanism. Annu Rev Pharmacol Toxicol. 2005;45:629-56).

In recent years the technology based on analysis of methylated DNA has come to be regarded as a powerful tool for the diagnosis, treatment, and prognosis of disease, as well as in the fields of forensic medicine, pharmacogenetics, and epidemiological studies. The association between the hypomethylated state of DNA and cancer, and later, its relationship with hypermethylation, have been known about since 1983; however, in the past five years, under the impetus of the new molecular strategies for studying de novo methylation of CpG islands, the analysis of methylated DNA has become a powerful biomarker for the early detection of cancer; in addition, it allows cancers to be classified according to histological subtypes, the degree of malignancy, differences in treatment response, and the various prognoses. An important recent application is precisely its use as a biomonitor of treatment response and a predictor of the prognosis in cancer.

DNA methylation is an epigenetic marker of gene silencing with applications in various fields of genetic and biomedical research which, through the application of molecular methodological processes, allows individual CpG island methylation patterns to be differentiated. Moreover, the methylation characteristics of the genes involved in neoplasia allow cancers to be classified and prognosed, and treatment to be followed up.

The development of DNA microarrays (also called DNA chips or microarrays), has made it possible for them quickly to be incorporated into genomic studies, making higher levels of resolution and sensitivity attainable in the comparative study of genomic DNA and a greater reproductive capacity, allowing reliable detection of changes in the genes at individual level. Thanks to its versatility, DNA microarray technology offers applications in the fields of transcriptomics, genetics, and epigenetics.

Hatada et al., "A microarray-based method for detecting methylated loci", Journal of Human Genetics-2002, vol. 47, pp. 448-451, disclosed a microarray-based method called methylation amplification DNA chip (MAD), which comprises the steps of digesting the DNA with the methylation-sensitive Smal enzyme and the methylation-insensitive Xmal enzyme, ligating with adaptor, PCR amplifying, Cy3/Cy5-labelling, and co-hybridizing to a microarray. The method allows the specific amplification of the CpG islands, leading to amplicons useful for hybridization, and resulting in high sensitivity. Hatada et al., propose the use of the method for detecting genes or tumor suppressor genes whose expression levels are too low to be found by an expression-based method.

### SUMMARY OF THE INVENTION

The present invention relates to a method of nucleic acid analysis comprising DNA The present invention relates to a method of nucleic acid analysis comprising DNA fragmentation, ligation of specific adaptors, an amplification stage, and labeling of samples using RNA polymerase. In this stage, a set of RNA fragments is generated, which are representative of the DNA fragments to be analyzed. These RNA fragments can, for example, later be hybridized using a DNA microarray to carry out the analysis. The method of the present invention can be used for selectively identifying methylation events in the analyzed samples.

In one embodiment, the invention provides a method for determining the methylation of a nucleic acid. The method of this embodiment comprises providing or obtaining a sample having a nucleic acid, treating the nucleic acid sample with a methylation insensitive restriction enzyme, treating the sample with a methylation sensitive restriction enzyme, ligating an adaptor to the site created by the methylation insensitive restriction enzyme, ligating an adaptor to the site create by the methylation sensitive restriction enzyme, subjecting the adaptor ligated nucleic acids to amplification conditions, labeling the amplified nucleic acid by in vitro transcription, and detecting the labeled nucleic acid.

In another embodiment, the invention provides a method for determining the methylation of a nucleic acid. The method of this embodiment comprises (1) providing or obtaining a sample having a nucleic acid, (2) treating the nucleic acid sample with a methylation insensitive restriction enzyme, (3) treating the sample with a methylation sensitive restriction enzyme, (4) ligating an adaptor to the site created by the methylation insensitive restriction enzyme, (5) ligating an adaptor to the site create by the methylation sensitive restriction enzyme wherein said adaptor is engineered to have a promoter suitable for in vitro transcription, (6) subjecting the adaptor ligated nucleic acids to amplification conditions, (7) labeling the amplified nucleic acid by in vitro transcription based on the promoter sequence in the adaptor ligated to the site created by the methylation sensitive enzyme, and (8) detecting the labeled nucleic acid. In some aspects of this embodiment, the amplification conditions are PCR amplification conditions. According to one aspect of this method, treatment of a nucleic acid that is methylated at the restriction site for the methylation sensitive restriction endonuclease yields larger fragments of DNA as compared to the non-methylated equivalent nucleic acid. The larger fragments are less likely to be amplified. In some aspects of this embodiment, the labeled nucleic acids are detected on a microarray.

### BRIEF DESCRIPTION OF THE DRAWINGS

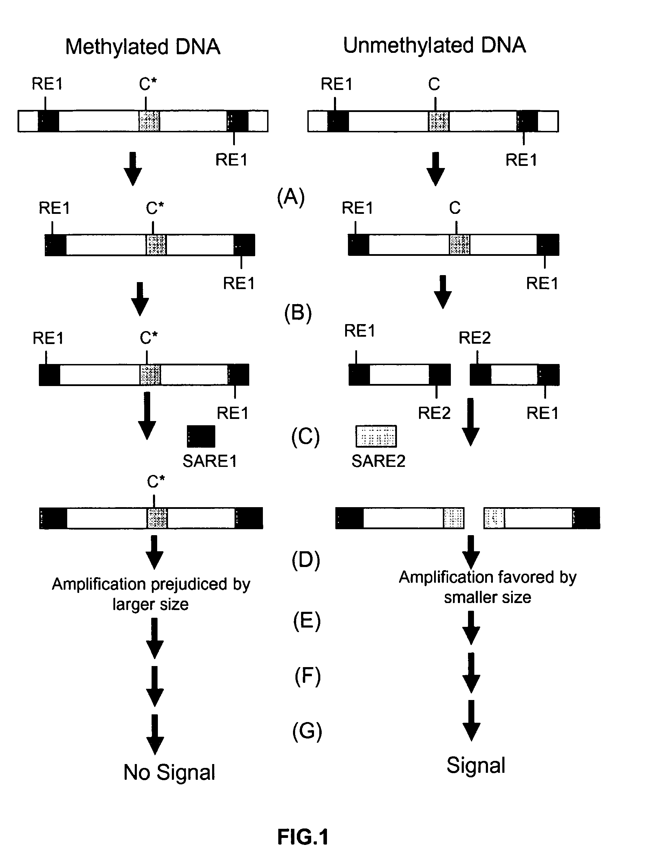

FIG. 1 shows a detailed diagram of the stages of one example of the method of the present invention. Step (A) refers to treatment with a methylation insensitive restriction enzyme (RE1). Step (B) refers to treatment with a methylation sensitive restriction enzyme (RE2). Step (C) refers to ligation of an adaptor specific for the site created by RE1 (SARE1) and an adaptor specific for the site create by RE2 (SARE2). In this example SARE2 has a sequence for an RNA polymerase promoter. Step (D) refers to PCR amplification. Step (E) refers to labeling by in vitro transcription. According to this example, the methylated sample does not end up being labeled since the fragment does not have the promoter for the RNA polymerase whereas the methylated sample has the SARE2 adaptors ligated to the fragment which has the promoter. Step (F) refers to hybridization. Step (G) refers to detection. The C* refers to a methylated cytosine in the methylated DNA sample whereas the "C" in the unmethylated sample represents an unmethylated cytosine. Treatment with RE2 cuts at the unmethylated cytosine, but not the methylated cytosine.

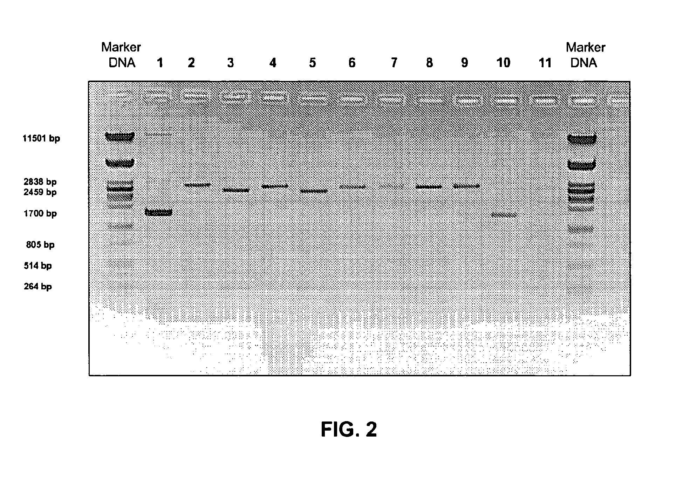

FIG. 2 shows the results of the digestion of pUC18 plasmid DNA and later ligation of adaptors as described in Example 1. The lane distribution is as follows:
   Lane 1 - 50 ng of undigested pUC18 plasmid (2686 bp).
   Lane 2 - 50 ng of pUC18 plasmid + Ndel enzyme (giving a linear band of 2686 bp). Tube 1.
   Lane 3 - 50 ng of digestion Tube 1 + unmethylated + TspMI enzyme (giving a band of 2435 bp + another band of 251 bp).
   Lane 4 - 50 ng of pUC18 plasmid + Ndel enzyme (giving a linear band of 2686 bp). Tube 2.
   Lane 5 - 50 ng of digestion Tube 2 + unmethylated + TspMI enzyme (giving a band of 2435 bp + another band of 251 bp).
   Lane 6 - 50 ng of pUC18 plasmid + Ndel enzyme (giving a linear band of 2686 bp). Tube 3.
   Lane 7 - 50 ng of digestion Tube 3 + methylated with Sssl methylase + TspMI enzyme (no modification of the 2686 bp band).
   Lane 8 - 50 ng of pUC18 plasmid + Ndel enzyme (giving a linear band of 2686 bp). Tube 4.
   Lane 9 - 50 ng of digestion Tube 4 + methylated with SssI methylase + TspMI enzyme (no modification of the 2686 bp band).
   Lane 10 - Ndel digestion negative control, i.e. pUC18 without Ndel enzyme (giving an original pUC18 profile with extra band due to the supercoiled form of the plasmid). Tube 5. Lane 11 - Ndel digestion negative control + TspMI enzyme. Tube 5 + digestion with TspMI enzyme (giving a linear band of 2686 bp).

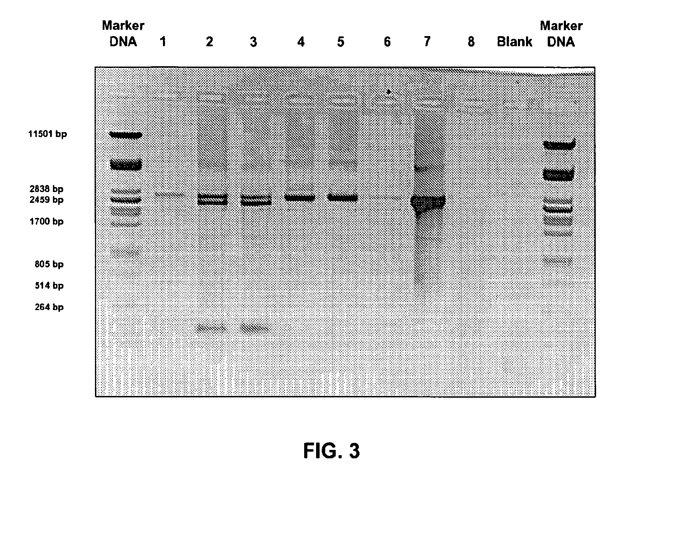

FIG. 3 shows the results of the amplification of pUC18 plasmid DNA as described in Example 1. The lane distribution is as follows:
   Lane 1 - PCR of Tube 1 without primers digested with ligated Ndel+TspMI. Negative (non-amplification) control.
   Lane 2 - PCR of Tube 1 digested with NdeI+TspMI - unmethylated and ligated (giving a band of 2435 bp + another band of 251 bp).
   Lane 3 - PCR of Tube 2 digested with NdeI+TspMI - unmethylated and ligated (giving a band of 2435 bp + another band of 251 bp).
   Lane 4 - PCR of Tube 3 digested with NdeI+TspMI - methylated and ligated (giving a linear band of 2686 bp).
   Lane 5 - PCR of Tube 4 digested with NdeI+TspMI - methylated and ligated (giving a linear band of 2686 bp).
   Lane 7 - PCR positive control.
   Lane 8 - PCR negative control.

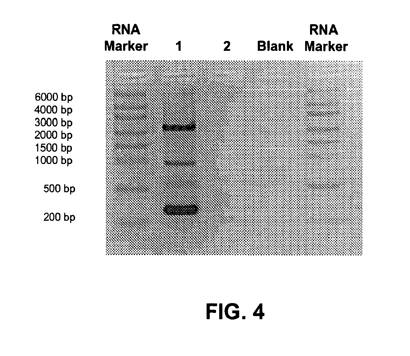

FIG. 4 shows the results of the in vitro transcription stage, as described in Example 1. The lane distribution is as follows:
   Lane 1 - In vitro transcription resulting from Tube 2 (plasmid digested with NdeI+TspMI, unmethylated). Resulting two principal bands correspond to the PCR-amplified bands of 2435 bp + the band of 251 bp. The presence of another two bands of approximately 900 bp and 500 bp could be explained as nonspecific bands produced by the PCR or else as artifacts of a concatenation of the small 251 bp plasmid fragment.
   Lane 2 - In vitro transcription resulting from Tube 2 (plasmid digested with NdeI+TspMI, methylated). It may be observed that in this lane there is no labeled RNA is present when the sample is methylated, as there was no TspMI cleavage and therefore no ligation occurred, nor was there any PCR product containing the T7 promoter.

### DESCRIPTION OF THE INVENTION

The present invention relates to the discovery of methods and compositions useful for analysis of nucleic acids. The method is useful for characterizing the methylation status or methylation profile of DNA. The compositions of the invention can be used for assessing the DNA methylation of genomic DNA. The method is useful in numerous applications including the diagnosis and prognosis of diseases having altered DNA methylation patterns. The method of the invention is also useful for biomarker discovery. The invention can be used to identify specific biomarkers associated with phenotypes and for establishing methylation fingerprints (e.g., patterns, status, profiles, or the methylome). Methylation patterns, status, profiles, and the methylome as determined by the methods of the invention can be associated with phenotypes (prognosis, diagnosis, response to therapeutics etc.). The method and compositions of the invention are useful for determining genome-wide methylation patterns.

In one embodiment, the present invention provides a method for determining the methylation of a nucleic acid comprising the following stages:
a) fragmentation of a genomic DNA sample with at least one methylation sensitive restriction enzyme and at least one methylation insensitive restriction enzyme,
b) ligation of specific adaptors to the ends of the DNA fragments obtained, where one of the specific adaptors comprises a functional promoter sequence,
c) amplification of the fragments that include both adaptors using specific primers based on the adaptors,
d) labeling of the amplified DNA fragments by in vitro transcription with an RNA polymerase capable of initiating transcription from the promoter sequence contained in one of the adaptors using a mixture of nucleotides, and
e) determining if the sample is methylated.

In an embodiment of the invention, fragmentation of a genomic DNA sample is achieved by digestion firstly with at least one methylation-insensitive restriction enzyme and then with at least one methylation-sensitive restriction enzyme.

In an embodiment of the invention, fragmentation of a genomic DNA sample is achieved by digestion firstly with at least one methylation-sensitive restriction enzyme and then with at least one methylation-insensitive restriction enzyme.

In an embodiment of the invention, fragmentation of a genomic DNA sample is achieved by digestion with at least one methylation-insensitive restriction enzyme and simultaneously with at least one methylation-sensitive restriction enzyme.

In an embodiment of the invention, the methylation-insensitive restriction enzyme recognizes a restriction enzymes target of 4, 5, or 6 base pairs.

In an embodiment of the invention, the methylation-insensitive restriction enzyme is selected from among the group comprising Bfal, TaqI, MseI, and NdeI.

In an embodiment of the invention, the methylation-sensitive restriction enzyme recognizes a restriction enzymes target of 4, 5, or 6 base pairs.

In an embodiment of the invention, the methylation-sensitive restriction enzyme is selected from among the group comprising Smal, Paul, TspMI, BsePI, and BssHII.

In an embodiment of the invention, the specific adaptor that comprises a functional promoter sequence is the specific adaptor for the methylation-sensitive restriction enzyme.

In an embodiment of the invention, labeling includes incorporation of nucleotide analogs containing directly detectable labeling substances, such as fluorophores, nucleotide analogs incorporating labeling substances detectable in a subsequent reaction, such as biotin or haptenes, or any other type of nucleic acid labeling.

In an embodiment of the invention, the nucleotide analog is selected from among the group comprising Cy3-UTP, Cy5-UTP, fluorescein-UTP, biotin-UTP, and aminoallyl-UTP.

The term functional promoter sequence refers to a sequence of nucleotides that can be recognized by an RNA polymerase and from which transcription can be initiated. In general, each RNA polymerase recognizes a specific sequence, so that the functional promoter sequence included in the adapters is chosen according to the RNA polymerase used. Examples of RNA polymerases that can be used in the method of the present invention include, but are not limited to, T7 RNA polymerase, T3 RNA polymerase, and SP6 RNA polymerase.

Determination of the methylation state of the sample can be performed using any nucleic acid analysis technique.

In an embodiment of the invention, determination of the methylation state of the sample is carried out by hybridization of the RNA fragments obtained in stage d) with the immobilized oligonucleotides on a DNA microarray, detection of the labeling incorporated in the fragments to be analyzed, and quantitative comparison of the signal values of the hybridized fragments with the values of the reference signals.

In an embodiment of the invention, the immobilized oligonucleotides on the microarray are designed in such a way as to include the restriction target of the methylation-sensitive restriction enzyme.

In an embodiment of the invention, the immobilized oligonucleotides on the microarray are designed so that they are located within the restriction targets of the methylation-sensitive restriction enzyme.

The term microarray or DNA microarray refers to a collection of multiple immobilized oligonucleotides on a solid substrate, where each oligonucleotide is immobilized in a known position so that hybridization with each of the multiple oligonucleotides can be detected separately. The substrate can be solid or porous, planar or non-planar, unitary or distributed. DNA microarrays on which hybridization and detection can be performed can be manufactured using oligonucleotides deposited by any mechanism or using oligonucleotides synthesized in situ by photolithography or by any other mechanism.

It is also an object of the present invention to provide a kit comprising the reagents, enzymes, and additives required to carry out the method of nucleic acid analysis of the invention.

In one embodiment, the invention to provides a kit comprising, (a) a component for fragmenting a DNA sample, (b) a component for ligation of specific adaptors to the ends of the DNA fragments obtained, (c) one or more adaptors for ligating to the fragmented DNA wherein at least one of the specific adaptors comprises a functional promoter sequence, (d) a component for amplification of the fragments that include both adaptors using specific primers based on the adaptors, and (e) a component for labeling of the amplified DNA fragments by in vitro transcription with an RNA polymerase capable of initiating transcription from the promoter sequence contained in one of the adaptors using a mixture of nucleotides.

The component for fragmenting a DNA sample comprises one or more endonucleases. In one aspect, the component for fragmenting the DNA comprises a methylation sensitive restriction endonuclease and a methylation insensitive restriction endonuclease. In one aspect, the methylation sensitive restriction endonuclease is selected from the group consisting of Smal, Paul, TspMI, BsePI, and BssHII. In one aspect, the methylation insensitive restriction endonuclease is selected from the group consisting of Mspl, Taql, Xmal, and FspBI.

The component for ligation of specific adaptors to the ends of the DNA fragments comprises a ligase. In one aspect, the ligase is T4 DNA ligase.

The one or more adaptors for ligating to the fragmented DNA are wherein at least one of the specific adaptors comprises a functional promoter sequence and wherein the adaptors are designed to hybridized to the cohesive ends created by the fragmentation of the DNA.

The component for amplification of the fragments can be used to amplify the fragmented genomic DNA using specific primers based on the adaptors. In one aspect, the component for amplification comprises specific primers based on the sequence of adaptors. In one aspect, the component for amplification comprises a polymerase. In one aspect, the polymerase is Taq polymerase. In one aspect, the component for amplification comprises dNTPs. In one aspect, the component for amplification comprises specific primers based on the sequence of adaptors, dNTPs, and a polymerase.

The component for labelling of the amplified DNA fragments by in vitro transcription comprises a RNA polymerase capable of initiating transcription from the promoter sequence contained in one of the adaptors using a mixture of nucleotides.

Another object of the present invention is the use of the previously described method for analyzing the methylation pattern presented by the CpG islands in the analyzed sample.

It is also an object of the present invention to use of the previously described method for diagnosing a disease state.

In an embodiment of the invention, the disease state is cancer. In another embodiment of the invention, the disease state is a neurodegenerative disease.

The sample of DNA (or nucleic acid) for use in the invention can be from any source and/or organism. For example, the DNA can be from human cells, human cancer cells, cancer cell lines, mammalian cells, mammalian cancer cells, mouse cells, cancer cells obtained from mice, plant cells, etc. The sample of DNA can also be obtained by various methods, e.g., from a biopsy, blood sample, aspirate, tissue section, a fluid sample, swab, etc.

The invention allows for the determination of the methylation profile of the genome of a cell or group of cells. The methylation profile of a cell, tissue or fluid can be correlated with specific phenotypic information and/or compared to "normal" methylation profiles. The methylation profile can also be used for diagnostic and/or prognostic information.

The method of the present invention is based on digestion of the genomic material with restriction enzymes (RE) and introduction of specific adaptors for the cleavage points. By selecting pairs of RE, for fragments generated with a combination of both RE, if one of the adaptors includes the splicing (i.e., promoter) sequence of an RNA-polymerase, it will be possible to transcribe this fragment in vitro for linear amplification and labeling.

In light of this approximation, we can put forward the following situation: RE2 is the enzyme for which an adaptor will be used that comprises the splicing (i.e., promoter) sequence of an RNA-polymerase and RE1 is the second enzyme whose adaptor does not contain the promoter sequence of an RNA-polymerase. Following the fragmentation, ligation, and amplification stages, only fragments that include segment RE2 (RE1-RE2; RE2-RE1; RE2-RE2) are capable of being amplified and labeled by in vitro transcription. Fragments RE1-RE1 are capable of being amplified but cannot be labeled by in vitro transcription (they do not have a splicing (i.e., promoter) site for RNA-polymerase) and will not create final material.

FIG. 1 shows a diagram of an example of the method of the present invention, indicating the stages that make up the said example.

In stage c) of the method of the present invention, preferably DNA fragments coming from fragments digested by both methylation-sensitive and methylation-insensitive enzymes and having fragments of statistically smaller size are amplified, and only the fragments that have incorporated the adaptor with the promoter will be labeled. This represents an advantage over other methods known in the state of the art, for example such as that described in WO2006088978, in which fragments of statistically larger size are amplified, i.e. fragments insensitive to digestion with the methylation-insensitive enzyme, which can reduce the effectiveness and reliability of the amplification.

Using the method of the present invention it is possible to obtain as final product a plurality of labeled RNAs, which can in their turn constitute the sample that can later be hybridized using a DNA microarray, which presents certain advantages compared to other methods. In the first place, the RNA-DNA interaction is stronger than the DNA-DNA interaction, enabling an increased average signal intensity to be obtained. In the second place, the single-stranded RNA does not face any competition from complementary molecules present in solution for hybridization on the microarray, so that a greater degree of hybridization is obtainable with the oligonucleotides on the surface of the DNA microarray.

As used herein, the term "methylation profile" refers to a set of data representing the methylation states of one or more loci within a molecule of DNA from e.g., the genome of an individual or cells or tissues from an individual. The profile can indicate the methylation state of every base in an individual, can have information regarding a subset of the base pairs (e.g., the methylation state of specific promoters or quantity of promoters) in a genome, or can have information regarding regional methylation density of each locus.

As used herein, the term "methylation status" refers to the presence, absence and/or quantity of methylation at a nucleotide or nucleotides within a portion of DNA. The methylation status of a particular DNA sequence can indicate the methylation state of every base in the sequence or can indicate the methylation state of a subset of the base pairs (e.g., whether the base is cytosine or 5-methylcytosine) within the sequence. Methylation status can also indicate information regarding regional methylation density within the sequence without specifying the exact location.

As used herein, the term "ligation" refers to any process of forming phosphodiester bonds between two or more polynucleotides, such as those comprising double stranded DNAs. Techniques and protocols for ligation may be found in standard laboratory manuals and references. Sambrook et al., In: Molecular Cloning. A Laboratory Manual 2nd Ed.; Cold Spring Harbor Laboratory Press, Cold Spring Harbor, N.Y. (1989) and Maniatis et al., pg. 146.

As used herein, the term "probe" refers to any nucleic acid or oligonucleotide that forms a hybrid structure with a sequence of interest in a target gene region (or sequence) due to complementarily of at least one sequence in the probe with a sequence in the target region.

As used herein, the terms "nucleic acid," "polynucleotide" and "oligonucleotide" refer to nucleic acid regions, nucleic acid segments, primers, probes, amplicons and oligomer fragments. The terms are not limited by length and are generic to linear polymers of polydeoxyribonucleotides (containing 2-deoxy-D-ribose), polyribonucleotides (containing D-ribose), and any other N-glycoside of a purine or pyrimidine base, or modified purine or pyrimidine bases. These terms include double- and single-stranded DNA, as well as double- and single-stranded RNA. A nucleic acid, polynucleotide or oligonucleotide can comprise, for example, phosphodiester linkages or modified linkages including, but not limited to phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages.

As used herein, the term "CpG Island", refers to any DNA region wherein the GC composition is over 50% in a "nucleic acid windows" having a minimum length of 200 bp nucleotides and a CpG content higher than 0.6.

As used herein, the term "promoter", refers to a sequence of nucleotides that resides on the 5' end of a gene's open reading frame. Promoters generally comprise nucleic acid sequences which bind with proteins such as, but not limited to, RNA polymerase and various histones.

In some embodiments, the methylation status of at least one cytosine, CpG island, or promoter is compared to the methylation status of a control locus. In some embodiments, the control locus is an endogenous control (e.g., comparison of tumor tissue to healthy tissue of the same origin as the tumor). In some embodiments, the control locus is an exogenous control (e.g., comparison of DNA from tissue of one individual to the DNA from the same tissue from a different individual).

In some aspects of the invention, the methylation status of normal tissue is compared to the methylation status of disease tissue. Several variants of these comparisons can be employed with the method of the invention, including comparing normal tissue from a group of subjects to matched disease tissue from a group of patients. For example, the methylation status of prostate cancer tissue obtained from patients having prostate cancer can be compared to normal non-cancerous prostate tissue (either derived from the sample population of patients and/or from healthy patients). Another example can use other tissue besides the diseased tissue: skin macrophages from healthy patients compared to skin macrophages from patients having disease (e.g., lung cancer). With a suitable sample size and sufficient experimental design, changes in the methylation status between the normal and diseased groups can identify biomarkers correlated with the characterisitic of interest (e.g., diagnosis, prognosis, likelihood of response to a therapeutic, etc.). The invention therefore allows for the determination of the methylation profile of the genome of a cell or group of cells. The methylation profile of a cell, tissue or fluid can be correlated with specific phenotypic information and/or compared to "normal" methylation profiles to identified patterns or specific markers associated with particular phenotypic information.

In yet another embodiment, the present invention provides methods for diagnosing or predicting a cancer by genome-wide methylation profiling. The method of this embodiment can comprise (1) obtaining a test sample from cells or tissue, (2) obtaining a control sample from cells or tissue that is normal, and (3) detecting or measuring in both the test sample and the control sample the genome-wide methylation profile using the method of the invention. If the methylation profile of test sample is altered compared to the control sample (or value), this indicates a cancer or a precancerous condition in the test sample cells. If the level methylation of one or more tumor suppressors is higher in the test sample as compared to the control sample (or value), this indicates a cancer or a precancerous condition in the test sample cells or tissue. If the level methylation of one or more oncogenes (e.g., genes whose higher expression imparts a more neoplastic or cancerous phenotype (such as EGFR)) is lower in the test sample as compared to the control sample (or value), this indicates a cancer or a precancerous condition in the test sample cells or tissue. In another aspect the control sample may be obtained from a different individual or be a normalized value based on baseline data obtained from a population.

In one embodiment, the method of the invention is used to determine whether two or more tumors are more likely to have arisen independently or more likely to be clonal (e.g., primary and metastasis). According to this method the methylation profiles determined by the method of the invention are compared. Methylation profiles that are substantially similar indicate that the tumors are more likely to be clonal whereas methylation profiles that are substantially different are more likely to have originated independently.

In yet another embodiment, the method of the invention providea a method of nucleic acid analysis. According to this embodiment, DNA is extracted or provided. In one specific aspect of this embodiment, the DNA is extracted by homogenizing tissue under cold conditions so that the DNA is not degraded (e.g., these conditions are achieved using liquid nitrogen (-180°C) and with continuous refrigeration of the mortar and tissue in use). Next, the homogenized tissue is resuspended in a DNA extraction buffer (e.g., 100 mM Tris-HCl; 50 mM EDTA pH 8; 500 mM NaCl)). In some aspects of this embodiment, the resuspended tissues brought to 65°C. In some aspects, the sample is treated to destroy or remove RNA (e.g., RNase A is added). In some aspects of this method, the resuspended tissue is treated with an agent that destroys or removes protein (e.g., 20 µl of Proteinase K and SDS is added). In some aspects, a solvent is then added to the resuspended sample (e.g., phenol-chloroform is added). Next, the DNA can be precipitated (e.g., addition of sodium acetate and 100% ethanol). In some aspects the precipitate is washed and then dissolved (e.g., in 50 µl of sterile water). The result of these steps is to provide DNA of sufficient purity to proceed with the remaining steps of this embodiment. As the skilled artisan recognizes, other methods or variants of these methods can yield DNA of sufficient purity to proceed with the remaining steps of the method.

Next, the DNA is digested with a methylation insensitive endocuclease (e.g., Bfal) and a methylation sensitive endocuclease (e.g., TspMI) either sequential (either treatment can be first) or at the same time. Next, the DNA fragments generated during the digestion are ligated to adaptors (e.g., Bfal adaptor compatible with the cohesive end of the BfaI enzyme and the TspMI adaptor compatible with the cohesive end of the TspMI) with a ligase (e.g., T4 DNA ligase). As the skilled artisan recognizes, other restrictions enzymes and adaptors can be substituted for those described above.

The adaptor ligated fragments are then amplified (e.g., the BfaI/TspMI fragments are amplified by PCR using two specific primers based on the sequence of adaptors).

The PCR-amplified DNA is then subjected to conditions sufficient for in vitro transcription (e.g., in vitro transcription to RNA based on a promoter sequence contained in the adaptor for the methylation sensitive endonuclease). This reaction can be carried out in duplicate using differently labeled nucleotides.

The resulting nucleic acids can then be detected (e.g., hybridized to a nucleic acid microarray).

In some embodiments, the method comprises determining the methylation status of at least 1, 2, 3, 4, 5, 6, 7, 8, 9, 10, 15, 20, 25, 30, 35, 40, 50, 75, or 100, 150, 200, 250, 300, 400, 500, 750, or 1000 cytosines in a DNA sample.

In some embodiments, the method comprises determining the methylation status of at least 1, 2, 3, 4, 5, 6, 7, 8, 9, 10, 15, 20, 25, 30, 35, 40, 50, 75, or 100, 150, 200, 250, 300, 400, 500, 750, or 1000 promoters in a DNA sample.

In some embodiments, the method comprises determining the methylation status of at least 1, 2, 3, 4, 5, 6, 7, 8, 9, 10, 15, 20, 25, 30, 35, 40, 50, 75, or 100, 150, 200, 250, 300, 400, 500, 750, or 1000 CpG islands within a DNA sample.

In one embodiment, the invention provides a microarray for determining the methylation status of at least 1, 2, 3, 4, 5, 6, 7, 8, 9, 10, 11, 12, 13, 14, 15, 20, 25, 30, 35, 40, 45, or 50 tumor suppressor promoters. According to this embodiment, the microarray is designed to have probes for determining the methylation status (or profile) of each promoter for each tumor suppressor, according to the method of the invention.

In one embodiment, the invention provides a microarray for determining the methylation status of at least 1, 2, 3, 4, 5, 6, 7, 8, 9, 10, 11, 12, 13, 14, 15, 20, 25, 30, 35, 40, 45, or 50 oncogenes. According to this embodiment, the microarray is designed to have probes for determining the methylation status (or profile) of each promoter for each tumor oncogene, according to the method of the invention.

The practice of the present invention employs, unless otherwise indicated, conventional techniques of chemistry, molecular biology, microbiology, recombinant DNA, genetics, immunology, cell biology, cell culture and transgenic biology, which are within the skill of the art. See, e.g., Maniatis, T., et al. (1982) Molecular Cloning: A Laboratory Manual (Cold Spring Harbor Laboratory, Cold Spring Harbor, N.Y.); Sambrook, J., et al. (1989) Molecular Cloning: A Laboratory Manual, 2nd Ed. (Cold Spring Harbor Laboratory, Cold Spring Harbor, N.Y.); Ausubel, F. M., et al. (1992) Current Protocols in Molecular Biology, (J. Wiley and Sons, NY); Glover, D. (1985) DNA Cloning, I and II (Oxford Press); Anand, R. (1992) Techniques for the Analysis of Complex Genomes, (Academic Press); Guthrie, G. and Fink, G. R. (1991) Guide to Yeast Genetics and Molecular Biology (Academic Press); Harlow and Lane (1988) Antibodies: A Laboratory Manual (Cold Spring Harbor Laboratory, Cold Spring Harbor, N.Y.; Jakoby, W. B. and Pastan, I. H. (eds.) (1979) Cell Culture. Methods in Enzymology, Vol. 58 (Academic Press, Inc., Harcourt Brace Jovanovich (NY); Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987).

### EXAMPLES

Below are described some non-exhaustive examples of the method of the present invention.

### Example 1: Analyzing the methylation state of a DNA fragment

### DNA digestion and adaptor ligation

The pUC18 plasmid DNA (500 ng) was digested overnight at 37°C with Ndel (methylation-insensitive enzyme) (Fermentas). The samples digested with Ndel were artificially methylated or not so as to simulate the actual situation of a DNA having methylated CpG. To methylate the samples, Sssl methylase enzyme (New England Biolabs) was used together with SAM (S-adenosyl methionine), incubating for 3 hours at 37°C. The representative unmethylated samples were kept at -20°C until the following enzymatic digestion step was performed with TspMI enzyme (methylation-sensitive enzyme) (New England Biolabs) for 3 hours at 75°C consecutively. To the DNA fragments generated following the last digestion were ligated the Ndel adaptor compatible with the cohesive end of the Ndel enzyme and the TspMI adaptor compatible with the cohesive end of the TspMI using T4 DNA ligase (Fermentas) in the T4 ligase buffer (Fermentas) incubated for 4 hours at room temperature.

The results are shown in FIG. 2.

### Amplification of DNA

The methylated/unmethylated samples digested with NdeI/TspMI were amplified by PCR using two specific primers based on the sequence of adaptors, to a concentration of 200 nM each in a reaction with 1x Taq, 1.5 mM of MgCl2, 200 nM of dNTP, 1 U of Taq polymerase (Fermentas) using the following cycle program: 72°C 2 min, 94°C 2 min, 35 cycles (94°C 30 sec, 58°C 30 sec, 72°C 3 min) and 72°C 10 min.

The results are shown in FIG. 3.

### In vitro transcription

2,5 µl of PCR-amplified DNA were used to carry out the in vitro transcription to RNA from a promoter sequence contained in the Sacl adaptor by the addition of 40 U of T7 RNA polymerase (Ambion) and 7.5 mM of rNTPS, incubating the samples for 1 hour 30 min at 37°C. This reaction was carried out in duplicate, in parallel with Cy3-dUTP or alternatively Cy5-dUTP (Perkin-Elmer) as labeled nucleotides. After transcription the labeled products were purified with MEGAclear™ columns (Ambion).

The results are shown in FIG. 4.

### Microarray hybridization

20 ng of unmethylated sample RNA labeled with Cy3 is combined with 20 ng of methylated sample RNA labeled with Cy5 to be hybridized to the microarray oligonucleotides. 100 µl of 2x hybridization solution (Agilent) is added to this RNA mixture, which is then loaded onto the chip as recommended by Agilent Technologies. Hybridization can be carried out overnight in a hybridization oven at 60°C: The microarray is subsequently washed with solutions 6x SSPE + 0.005% N-laurylsarcosine (SIGMA) at room temperature for 1 min while stirring, 0.06x of SSPE + 0.005% N-laurylsarcosine at room temperature for 1 min while stirring to remove any excess non-hybridized transcripts. Next, the chip is washed for 30 sec in a protective fluorophore solution containing acetonitrile and withdrawn from this solution slowly and at a constant speed to allow the chip to dry thoroughly and uniformly. The intensity signals of each nucleotide in the microarray are detected with an Agilent 62505B scanner.

### Example 2: Analyzing the methylation state of a sample of human DNA

### Preparing the DNA

Genomic DNA is extracted from a type of human tissue. The tissue is broken up and the cells are ground down in a cold porcelain mortar. This breaking-up of the tissue has to be performed under cold conditions otherwise the DNA can degrade, and these conditions are achieved using liquid nitrogen (-180°C) and with continuous refrigeration of the mortar and tissue in use. Once the tissue is homogenized it is resuspended in 600 µl of DNA extraction solution (100 mM Tris-HCl; 50 mM EDTA pH 8; 500 mM NaCl) preheating to 65°C. 2 µl of RNase A (10 mg/ml) is added and kept at 37°C for 15 min. After that, 20 µl of Proteinase K (20 mg/ml) + 50 µl of 20% SDS is added, mixed well, and incubated for 3 hours at 65°C. 1 volume of phenol-chloroform is added, the mixture is thoroughly homogenized for 5 min manually, and then centrifuged at 4°C and 13000 rpm for 5 minutes in a microcentrifuge. The supernatant is pipetted into a fresh tube and a further 1 volume of phenol-chloroform was added. The mixture is centrifuged for a further 5 min at 4°C and 13000 rpm. The supernatant is pipetted into another fresh tube, adding 1/10 volume of 5 M sodium acetate and 2 volumes of cold 100% ethanol. The sample tubes are left for 1 hour at -20°C for the DNA to precipitate. After the lapse of this period of time the DNA is precipitated out by centrifuging at 13000 rpm for 30 min at 4°C, the precipitate is washed with 500 µl of 70% ethanol and left to dry. The precipitate is dissolved in 50 µl of sterile water.

### DNA digestion and adaptor ligation

The total amount (2 µg) of genomic DNA is digested with BfaI (insensitive enzyme) (Fermentas) and TspMI (enzyme sensitive to methyl groups) (New England Biolabs) for an incubation time of 3 hours at 37°C followed consecutively by 3 hours at 75°C. To the DNA fragments generated following the digestion there are ligated the BfaI adaptor compatible with the cohesive end of the Bfal enzyme and the TspMI adaptor compatible with the cohesive end of the TspMI with T4 DNA ligase (Fermentas, Lithuania) in the T4 ligase buffer (Fermentas, Lithuania) incubated for 4 hours at room temperature.

### Amplification of DNA

The BfaI/TspMI fragments are amplified by PCR using two specific primers based on the sequence of adaptors to a concentration of 200 nM each in a reaction with 1x Taq buffer, 1.5 mM of MgCl₂, 200 nM of dNTP, 1 U of Taq polymerase (Fermentas, Lithuania) using the following cycle program: 2 min at 72°C; 2 min at 94°C; 34 cycles of 30 sec at 94°C, 30 sec at 56°C, 90 sec at 72°C, and 10 min at 72°C.

### In vitro transcription

2,5 µg of PCR-amplified DNA are used to carry out the in vitro transcription to RNA from a promoter sequence contained in SacI adaptor by the addition of 40 U of T7 RNA polymerase (Ambion, USA) and 7.5 mM of rNTPS, incubating the samples overnight at 37°C. This reaction is carried out in duplicate, in parallel using Cy3-dUTP or else Cy5-dUTP (Perkin-Elmer, USA) as labeled nucleotides. After transcription, the DNA is removed by treating with 2 U of DNase I (Ambion, USA) at 37°C for 30 min. The labeled products are purified using MEGAclear™ columns (Ambion, USA).

### Microarray hybridization

0.75 µg of sample RNA labeled with Cy3 is combined with 0.75 µg of sample RNA labeled with Cy5 to be hybridized to the microarray oligonucleotides. 100 µl of 2 hybridization solution (Agilent, USA) is added to this RNA mixture and loaded onto the chip as recommended by the company Agilent Technologies. Hybridization takes place overnight in a hybridization oven at 60°C: The microarray is subsequently washed with solutions 6x SSPE + 0.005% N-laurylsarcosine (SIGMA) at room temperature for 1 min while stirring, and 0.06x of SSPE + 0.005% N-laurylsarcosine at room temperature for 1 min while stirring to remove any excess non-hybridized transcripts. Next, the chip is washed for 30 sec in a protective fluorophore solution containing acetonitrile and withdrawn from this solution slowly and at a constant speed to allow the chip to dry thoroughly and uniformly. The intensity signals of each nucleotide in the microarray is detected with an Agilent 62505B scanner.

## Claims

1. A method for determining the methylation of a nucleic acid comprising the following stages:
a) fragmentation of a genomic DNA sample with at least one methylation sensitive restriction enzyme and at least one methylation insensitive restriction enzyme,
b) ligation of specific adaptors to the ends of the DNA fragments obtained, where one of the specific adaptors comprises a functional promoter sequence,
c) amplification of the fragments that include both adaptors using specific primers based on the adaptors,
d) labeling of the amplified DNA fragments by in vitro transcription with an RNA polymerase capable of initiating transcription from the promoter sequence contained in one of the adaptors using a mixture of nucleotides, and
e) determining if the sample is methylated.

2. The method of claim 1, wherein fragmentation of a genomic DNA sample is achieved by digestion in the first place with at least one methylation-insensitive restriction enzyme and subsequently with at least one methylation-sensitive restriction enzyme; or , wherein fragmentation of a genomic DNA sample is achieved by digestion in the first place with at least one methylation-sensitive restriction enzyme and subsequently with at least one methylation-insensitive restriction enzyme; or wherein fragmentation of a genomic DNA sample is achieved by digestion with at least one methylation-insensitive restriction enzyme and simultaneously with at least one methylation-sensitive restriction enzyme.

3. The method claim 1, wherein the methylation-insensitive restriction enzyme recognizes a restriction enzymes target of 4, 5, or 6 base pairs.

4. The method of claim 3, wherein the methylation-insensitive restriction enzyme is selected from the group comprising Bfal, Taql, Msel, and Ndel.

5. The method of claim 1, wherein the methylation-sensitive restriction enzyme recognizes a restriction enzymes target of 4, 5, or 6 base pairs.

6. The method of claim 5, wherein the methylation-sensitive restriction enzyme is selected from the group comprising Smal, Paul, TspMI, BsePI, and BssHII.

7. The method of claim 1, wherein the specific adaptor comprising a functional promoter sequence is the specific adaptor for the methylation-sensitive restriction enzyme.

8. The method of claim 1, wherein the labeling comprises the incorporation of nucleotide analogs containing directly detectable labeling substances, such as fluorophores, nucleotide analogs incorporating labeling substances detectable in a subsequent reaction, such as biotin or haptenes, or any other type of nucleic acid labeling substances.

9. The method of claim 1, wherein the RNA polymerase is selected from the group comprising T7 RNA polymerase, T3 RNA polymerase, and SP6 RNA polymerase.

10. The method of claim 1, wherein the determination of the methylation state of the sample is carried out by hybridization of the RNA fragments obtained in stage d) with the immobilized oligonucleotides on a DNA microarray, detection of the labeling incorporated in the fragments to be analyzed, and quantitative comparison of the signal values of the hybridized fragments with the values of the reference signals.

11. The method of claim 10, wherein the immobilized oligonucleotides on the microarray are designed in such a way as to include the restriction target of the methylation-sensitive restriction enzyme; or wherein the immobilized oligonucleotides on the microarray are designed in such a way that they are located within the restriction targets of the methylation-sensitive restriction enzyme.

12. A kit comprising the reagents, enzymes, and additives required to carry out the method of any one of claims 1 to 11.

13. Use of the method claimed in any one of claims 1 to 11 for analyzing the methylation pattern presented by the CpG islands in the analyzed sample.

14. Use of the method claimed in any one of claims 1 to 11 for the diagnosis of a disease state.

15. Use of the method as claimed in claim 14, wherein the disease state is selected from the group consisting of cancer and a neurodegenerative disease.

## Patentansprüche

1. Verfahren zur Bestimmung der Methylierung einer Nukleinsäure mit den folgenden Phasen;
a) Fragmentierung einer genomischen DNS-Probe mit mindestens einem methylierungssensitiven Restriktionsenzym und mindestens einem methylierungsinsensitiven Restriktionsenzym.
b) Ligation spezifischer Adapter an die Enden der erhaltenen DNS-Fragmente, wobei einer der spezifischen Adapter eine funktionelle Promotorsequenz enthält,
c) Amplifikation der Fragmente, die beide Adapter enthalten, unter Anwendung spezifischer auf den Adaptern befindlicher Primer.
d) Markieren der amplifizierten DNS-Fragmente durch In-Vitro-Transkription mit einer RNS-Polymerase, die in der Lage ist, eine Transkription unter Verwendung einer Mischung aus Nukleotiden von der in einem der Adapter enthaltenen Promotorsequenz aus in Gang zu setzen, und
e) Bestimmen, ob die Probe methyliert ist.

2. Verfahren nach Anspruch 1, bei dem die Fragmentierung einer genomischen DNS-Probe **dadurch** erreicht wird, dass sie zunächst mit mindestens einem methylierungsinsensitiven Restriktionsenzym und anschließend mit mindestens einem methylierungssensitiven Restriktionsenzym verdaut wird; oder bei dem die Fragmentierung einer genomischen DNS-Probe **dadurch** erreicht wird, dass sie zunächst mit mindestens einem methylierungssensitiven Restriktionsenzym und anschließend mit mindestens einem methylierungsinsensitiven Restriktionsenzym verdaut wird; oder bei dem die Fragmentierung einer genomischen DNS-Probe **dadurch** erreicht wird, dass sie mit mindestens einem methylierungsinsensitiven Restriktionsenzym und gleichzeitig mit mindestens einem methylierungssensitiven Restriktionsenzym verdaut wird.

3. Verfahren nach Anspruch 1, bei dem das methylierungsinsensitive Restriktionsenzym ein Restriktionsenzymtarget aus 4, 5 oder 6 Basenpaaren erkennt.

4. Verfahren nach Anspruch 3, bei dem das methylierungsinsensitive Restriktionsenzym aus der Gruppe ausgewählt wird, die Bfal, Taql, Msel, und Ndel umfasst.

5. Verfahren nach Anspruch 1, bei dem das methylierungssensitive Restriktionsenzym ein Restriktionsenzymtarget aus 4, 5 oder 6 Basenpaaren erkennt.

6. Verfahren nach Anspruch 5, bei dem das methylierungssensitive Restriktionsenzym aus der Gruppe ausgewählt wird, die Smal, Paul, TspMI, BsePI, und BssHII umfasst.

7. Verfahren nach Anspruch 1, bei dem der spezifische Adapter, der eine funktionelle Promotorsequenz enthält, der spezifische Adapter für das methylierungssensitive Restriktionsenzym ist.

8. Verfahren nach Anspruch 1, bei dem das Markieren das Einbauen von Nukleotidanaloga, die unmittelbar erkennbare Markersubstanzen wie zum Beispiel Fluorophore enthalten, Nukleotidanaloga mit Markersubstanzen, die in einer nachfolgenden Reaktion erkennbar sind, wie zum Beispiel Biotin oder Haptene, oder irgendeine andere Art von Nukleinsäure-Markersubstanzen umfasst.

9. Verfahren nach Anspruch 1, bei dem die RNS-Polymerase aus der Gruppe ausgewählt wird, die T7-RNS-Polymerase, T3-RNS-Polymerase und SP6-RNS-Polymerase umfasst.

10. Verfahren nach Anspruch 1, bei dem die Bestimmung des Methylierungszustandes der Probe mittels Hybridisierung der in Phase d) erhaltenen RNS-Fragmente mit immobilisierten Oligonukleotiden auf einem DNS-Mircoarray, Ausfindigmachen der in die Fragmente, die analysiert werden sollen, eingebauten Marker, und quantitativem Vergleich der Signalwerte der hybridisierten Fragmente mit den Werten der Referenzsignale durchgeführt wird.

11. Verfahren nach Anspruch 10, bei dem die immobilisierten Oligonukleotide auf den Microarrays so gestaltet sind, dass sie das Restriktionstarget der methylierungssensitiven Restriktionsenzyme enthalten; oder bei dem die immobilisierten Oligonukleotide auf dem Microarray so gestaltet sind, dass sie innerhalb der Restriktionstargets der methylierungssensitiven Restriktionsenzyme liegen.

12. Kit, das die Reagenzien, Enzyme und Zusatzstoffe, die zur Durchführung des Verfahrens gemäß einem der Ansprüche 1 bis 11 nötig sind, enthält.

13. Verwendung des Verfahrens nach einem der Ansprüche 1 bis 11 zur Analyse des Methylierungsmusters, das die CpG-Inseln in der analysierten Probe aufweisen.

14. Verwendung des Verfahrens nach einem der Ansprüche 1 bis 11 für die Diagnose eines Krankheitszustandes.

15. Verwendung des Verfahrens nach Anspruch 14, bei dem der Krankheitszustand aus der Gruppe ausgewählt wird, die aus Krebs und einer neurodegenerativen Krankheit besteht.

## Revendications

1. Procédé pour déterminer la méthylation d'un acide nucléique comprenant les phases suivantes :
a) une fragmentation d'un échantillon d'ADN génomique avec au moins une enzyme de restriction sensible à la méthylation et au moins une enzyme de restriction insensible à la méthylation,
b) une ligature d'adaptateurs spécifiques aux extrémités des fragments d'ADN obtenus, où l'un des adaptateurs spécifiques comprend une séquence de promoteur fonctionnelle,
c) une amplification des fragments qui comprennent l'un et l'autre adaptateur en utilisant des amorces spécifiques basées sur les adaptateurs,
d) un marquage des fragments d'ADN amplifiés par transcription in vitro avec une ARN polymérase susceptible d'initier une transcription à partir de la séquence de promoteur contenue dans un des adaptateurs en utilisant un mélange de nucléotides, et
e) une détermination si l'échantillon est méthylé.

2. Procédé selon la revendication 1, dans lequel la fragmentation d'un échantillon d'ADN génomique est obtenue par digestion au premier endroit avec au moins une enzyme de restriction insensible à la méthylation et par la suite avec au moins une enzyme de restriction sensible à la méthylation ; ou, dans lequel la fragmentation d'un échantillon d'ADN génomique est obtenue par digestion au premier endroit avec au moins une enzyme de restriction sensible à la méthylation et par la suite avec au moins une enzyme de restriction insensible à la méthylation ; ou, dans lequel la fragmentation d'un échantillon d'ADN génomique est obtenue par digestion avec au moins une enzyme de restriction insensible à la méthylation et simultanément avec au moins une enzyme de restriction sensible à la méthylation.

3. Procédé selon la revendication 1, dans lequel l'enzyme de restriction insensible à la méthylation reconnaît une cible d'enzymes de restriction de 4, 5 ou 6 paires de base.

4. Procédé selon la revendication 3, dans lequel l'enzyme de restriction insensible à la méthylation est choisie dans le groupe comprenant Bfal, Taql, Msel, et Ndel.

5. Procédé selon la revendication 1, dans lequel l'enzyme de restriction sensible à la méthylation reconnaît une cible d'enzymes de restriction de 4, 5 ou 6 paires de base.

6. Procédé selon la revendication 5, dans lequel l'enzyme de restriction sensible à la méthylation est choisie dans le groupe comprenant Smal, Paul, TspMI, BsePI, and BssHII.

7. Procédé selon la revendication 1, dans lequel l'adaptateur spécifique comprenant une séquence de promoteur fonctionnelle est l'adaptateur spécifique pour l'enzyme de restriction sensible à la méthylation.

8. Procédé selon la revendication 1, dans lequel le marquage comprend l'incorporation d'analogues de nucléotide contenant des substances de marquage directement détectables, telles que des fluorophores, des analogues de nucléotide incorporant des substances de marquage détectables dans une réaction ultérieure, telles que la biotine ou des haptènes, ou n'importe quel autre type de substances de marquage d'acide nucléique.

9. Procédé selon la revendication 1, dans lequel l'ARN polymérase est choisie dans le groupe comprenant l'ARN polymérase T7, l'ARN polymérase T3, et l'ARN polymérase SP6.

10. Procédé selon la revendication 1, dans lequel la détermination de l'état de méthylation de l'échantillon est effectuée par hybridation des fragments d'ARN obtenus dans la phase d) avec les oligonucléotides immobilisés sur une micromatrice d'ADN, la détection du marquage incorporé dans les fragments à analyser, et une comparaison quantitative des valeurs de signal des fragments hybridés avec les valeurs des signaux de référence.

11. Procédé selon la revendication 10, dans lequel les oligonucléotides immobilisés sur la micromatrice sont conçus d'une manière telle à inclure la cible de restriction de l'enzyme de restriction sensible à la méthylation ; ou dans lequel les oligonucléotides immobilisés sur la micromatrice sont conçus d'une manière telle qu'ils sont situés au sein des cibles de restriction de l'enzyme de restriction sensible à la méthylation.

12. Trousse comprenant les réactifs, enzymes, et additifs requis pour effectuer le procédé selon l'une quelconque des revendications 1 à 11.

13. Utilisation du procédé revendiqué dans l'une quelconque des revendications 1 à 11 pour analyser le schéma de méthylation présenté par les îlots CpG dans l'échantillon analysé.

14. Utilisation du procédé revendiqué dans l'une quelconque des revendications 1 à 11 pour le diagnostic d'un état maladif.

15. Utilisation du procédé selon la revendication 14, dans lequel l'état maladif est choisi dans le groupe constitué d'un cancer et une maladie neurodégénérative.
